(19) Europäisches Patentamt
European Patent Office
Office européen des brevets

(11) **EP 0 496 454 B1**

(12) **EUROPÄISCHE PATENTSCHRIFT**

(45) Veröffentlichungstag und Bekanntmachung des Hinweises auf die Patenterteilung:
**21.04.1999 Patentblatt 1999/16**

(51) Int Cl.6: **G01N 23/201**, G21K 1/02, A61B 6/02, A61B 6/03

(21) Anmeldenummer: **92200113.6**

(22) Anmeldetag: **16.01.1992**

(54) **Röntgengerät**

X-Ray apparatus

Appareil de radiographie

(84) Benannte Vertragsstaaten:
**DE FR GB**

(30) Priorität: **19.01.1991 DE 4101544**

(43) Veröffentlichungstag der Anmeldung:
**29.07.1992 Patentblatt 1992/31**

(73) Patentinhaber:
• **Philips Patentverwaltung GmbH**
**22335 Hamburg (DE)**
Benannte Vertragsstaaten:
**DE**
• **Koninklijke Philips Electronics N.V.**
**5621 BA Eindhoven (NL)**
Benannte Vertragsstaaten:
**FR GB**

(72) Erfinder:
• **Harding, Geoffrey, Dr.**
**W-2000 Hamburg 53 (DE)**
• **Martens, Gerhard, Dr.**
**W-2359 Henstedt-Ulzburg (DE)**

(74) Vertreter: **Hartmann, Heinrich, Dipl.-Ing. et al**
**Philips Patentverwaltung GmbH,**
**Röntgenstrasse 24**
**22335 Hamburg (DE)**

(56) Entgegenhaltungen:
EP-A- 0 209 952          GB-A- 1 463 054
US-A- 4 825 454

**Beschreibung**

Die Erfindung betrifft ein Röntgengerät mit einem polychromatischen Röntgenstrahler zur Erzeugung eines Primärstrahls von geringem Querschnitt, einer energieauflösend messenden Detektoranordnung zur Erfassung der durch elastische Streuprozesse im Primärstrahl erzeugten Streustrahlung mit mehreren Detektorelementen, die auf zum Primärstrahl konzentrischen Ringen angeordnet sind, einer zwischen dem Röntgenstrahler und der Detektoranordnung angeordneten, den Primärstrahl umschließenden Kollimatoranordnung und einer an die Detektoranordnung angeschlossenen Auswerteeinrichtung.

Ein solches Röntgengerät ist im wesentlichen aus der DE-OS 37 12 928 bekannt. Die Kollimatoranordnung ist dabei so gestaltet, daß die Detektorelemente unterschiedliche Abschnitte des Primärstrahls innerhalb eines Untersuchungsbereichs erfassen und daß ein Schichtbild der Streudichte herstellbar ist, wenn der Untersuchungsbereich vom Primärstrahl in unterschiedlichen Richtungen und längs paralleler Strahlenpfade durchstrahlt wird.

Aus der Zeitschrift "Phys. Med. Biol." (1990, Vol 35, No 1, S. 33-41) ist ein Röntgengerät bekannt, mit dem das Impulsübertragsspektrum eines kleinen Bereiches innerhalb eines menschlichen Körpers bestimmt werden kann. Da sich die Impulsübertragsspektren gesunder und kranker (von Osteoporose befallener) Knochen deutlich unterscheiden, erlaubt es ein solches Verfahren, den Zustand der Knochensubstanz zu beurteilen. Zur Erfassung des Impulsübertragsspektrums wird die unter einem festen Streuwinkel (z.B. 3,5°) im Primärstrahl gestreute Strahlung mittels eines energieauflösenden Detektors erfaßt und der Impulsübertrag bestimmt nach der Gleichung

$$X = \sin (T/2)/L, \qquad (1)$$

wobei T der Streuwinkel ist und L die Wellenlänge der Streustrahlung. Da die Streuquerschnitte für elastische Streustrahlung relativ gering sind, hat das bekannte Gerät eine vergleichsweise geringe Empfindlichkeit, so daß bei derartigen Untersuchungen relativ große Strahlungsdosen erforderlich sind. Es ist zwar möglich, die Strahlungsdosis dadurch zu vergrößern, daß der fest vorgegebene Winkelbereich vergrößert wird, innerhalb dessen die Streustrahlung erfaßt wird, doch wird dadurch die Genauigkeit verringert, mit der der Impulsübertrag bestimmt werden kann.

Aufgabe der vorliegenden Erfindung ist es, ein Röntgengerät der eingangs genannten Art so auszugestalten, daß das Impulsübertragsspektrum eines kleinen Volumenbereichs innerhalb eines ausgedehnten Objekts mit relativ geringer Dosis vergleichsweise genau bestimmbar ist.

Diese Aufgabe wird erfindungsgemäß durch die im Hauptanspruch angegebenen Maßnahmen gelöst.

Jedes der Detektorelemente erfaßt die Streustrahlung unter einem verhältnismäßig engen Streuwinkelbereich, so daß - bei hinreichendem Energieauflösungsvermögen der einzelnen Detektorelemente - das Impulsübertragsspektrum verhältnismäßig genau bestimmbar ist. Da mehrere Detektorelemente die Streustrahlung aus demselben Abschnitt erfassen und da das Impulsübertragsspektrum aus den Ausgangssignalen all dieser Detektorelemente abgeleitet wird, entspricht die Vergrößerung der Empfindlichkeit bzw. die Verringerung der Strahlungsdosis der Zahl der Detektorelemente, die denselben Abschnitt des Primärstrahls "sehen".

Es sei an dieser Stelle erwähnt, daß aus der EP-A 209 952 bereits ein Röntgengerät bekannt ist, bei dem mehrere Detektorelemente, die auf zu einem Primärstrahl konzentrischen Ringen angeordnet sind, durch eine Blendenplatte hindurch die Streustrahlung erfassen, die durch den Primärstrahl in einem Untersuchungsobjekt erzeugt wird. Jedes Detektorelement erfaßt dabei also die im gesamten Untersuchungsbereich erzeugte Streustrahlung und nicht nur die Streustrahlung aus einem kleinen Volumenbereich. Um das Impulsübertragsspektrum eines kleinen Untersuchungsbereichs zu ermitteln, ist ein aufwendiges in der DE-A 34 06 905 beschriebenes Verfahren erforderlich, das in der Praxis nicht zu befriedigenden Resultaten führt.

Weiterhin ist aus der GB-PS 1 463 054 bereits ein Röntgengerät bekannt, bei dem die in einem Primärstrahl erzeugte Streustrahlung von mehreren auf zum Primärstrahl konzentrischen Ringen angeordneten Detektorelementen erfaßt wird, wobei eine Kollimatoranordnung vorgesehen ist, die so gestaltet ist, daß die Detektorelemente von der Streustrahlung nur ein und desselben Abschnitts (7) des Primärstrahls getroffen werden. Die Primärstrahlung ist dabei so hart (zwischen 200 keV und 2 MeV), und die Streuwinkel, unter denen die Detektorelemente die Streustrahlung erfassen, sind so groß, daß im wesentlichen nur Compton-Streustrahlung erfaßt wird. Deshalb ist die Bestimmung des Impulsübertragsspektrums im Untersuchungsbereich mit der bekannten Anordnung nicht möglich, die darüberhinaus eine monochromatische Strahlenquelle voraussetzt.

Bevorzugte Weiterbildungen der Erfindung sind in den Unteransprüchen beschrieben.

Die Erfindung wird nachstehend anhand der Zeichnung näher erläutert.

Es zeigen

Fig. 1a ein erfindungsgemäßes Röntgengerät,

Fig. 1b eine spezielle Ausgestaltung eines Detektorelements,

Fig. 2 eine Einrichtung zur Bestimmung des Impulsübertragsspektrums aus den Ausgangssignalen der Detektorelemente und

Fig. 3 die Energiespektren verschiedener Detektorelemente und das daraus resultierende Impulsübertragsspektrum.

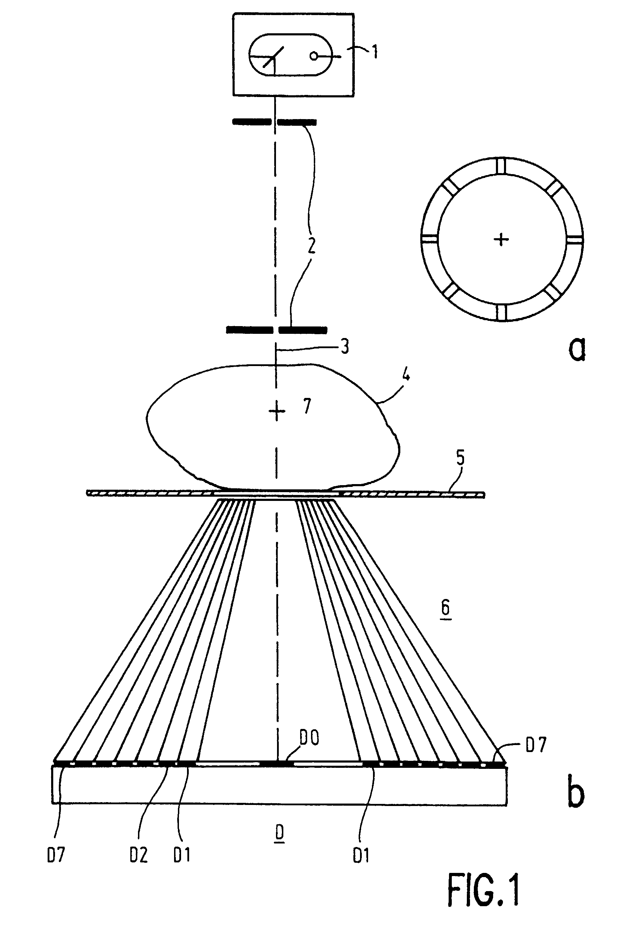

In Fig. 1a ist mit 1 ein polychromatischer Röntgenstrahler bezeichnet, der beispielsweise eine Röntgenröhre enthalten kann. Der polychromatische Röntgenstrahler 1 emittiert in einem Energiebereich, der im Falle einer Röntgenröhre von der daran anliegenden Hochspannung abhängt, Röntgenstrahlung. Mittels wenigstens einer Lochblende 2 wird von der erzeugten Röntgenstrahlung ein Nadelstrahl (pencil beam) 3, d.h. ein Primärstrahl mit geringem Querschnitt ausgeblendet. Dieser Primärstrahl durchsetzt ein ausgedehntes Objekt 4, beispielsweise einen Patienten, der auf einer Tischplatte 5 gelagert ist. Unterhalb der Tischplatte befindet sich eine Detektoranordnung D, die die Intensität des Primärstrahls und der im Primärstrahl erzeugten Streustrahlung mißt. Die Detektoranordnung mißt die Röntgenstrahlung energieaufgelöst, d.h. ihre Ausgangssignale hängen (linear) von der Energie der jeweils detektierten Röntgenquanten ab.

Die Detektoranordnung D umfaßt einen konzentrisch zum Primärstrahl 3 angeordneten kreisförmigen Germaniumkristall mit einem zentralen Detektorelement $D_0$ und konzentrisch dazu angeordneten ringförmigen Detektorelementen $D_1..D_7$., die die Röntgenquanten unabhängig voneinander detektieren. Das zentrale Detektorelement $D_0$ mißt die Intensität des Primärstrahls nach dem Durchsetzen des Körpers 4, während die Detektorelemente $D_1..D_7$ die Streustrahlung aus dem Untersuchungsbereich getrennt voneinander erfassen. Jedes Detektorelement ist 2 mm breit, und zwischen je zwei Detektorelementen befindet sich ein Zwischenraum von 0,5 mm. Das äußerste Detektorelement hat einen Außendurchmesser von 70 mm.

Zwischen der Tischplatte 5 und der Detektoranordnung D ist eine Kollimatoranordnung 6 vorgesehen. Diese besteht aus einer Anzahl von Kollimatorlamellen, die die Form des Mantels eines Kegelstumpfes aufweisen. Die Verlängerungen der Mantelflächen dieser Kollimatorlamellen schneiden sich in dem durch ein Kreuz markierten Punkt 7 innerhalb des Körpers 4. Es sind mindestens soviele Kollimatorlamellen vorgesehen wie Detektorelemente, wobei die Anordnung so getroffen ist, daß die Streustrahlung zwischen benachbarten Kollimatorlamellen jeweils nur auf eines der Detektorelemente $D_1..D_7$ auftreffen kann. Die Kollimatorlamellen bestehen aus Strahlen absorbierendem Material, beispielsweise aus 0,2 mm starken Blechen einer Kupfer/Silber-Legierung.

Da sich die Kollimatoren im Punkt 7 schneiden, "sehen" alle Detektorelemente nur diesen Punkt bzw. den Bereich des Primärstrahls um diesen Punkt herum. Die Streuwinkel der Streustrahlung, die die Detektorelemente $D_1..D_7$ erfassen, nehmen von innen ($D_1$), nach außen ($D_7$) zu.

Es sei an dieser Stelle bemerkt, daß zwecks besserer Darstellung die Zeichnung die tatsächlichen Größenverhältnisse nicht richtig wiedergibt. Die Höhe der Kollimatoren beträgt 300 mm, während der Durchmesser des äußersten Kollimators (auf seiner dem Detektor zugewandten Seite) 70 mm beträgt. Der Punkt 7 ist von der Detektoranordnung D 500 mm entfernt.

Bei diesen Abmessungen trifft nur Streustrahlung, die mit dem Primärstrahl einen Winkel (den Streuwinkel) zwischen zwei und vier Grad einschließt, auf die Detektorelemente $D_1..D_7$. In diesem Winkelbereich überwiegt bei einer Röhrenspannung von z.B. 160 kV die elastische bzw. kohärente Streustrahlung die Compton-Streustrahlung. Elastische Streustrahlung entsteht durch Streuprozesse, bei denen die Röntgenquanten ihre Energie nicht ändern.

Bekanntlich hängt das Impulsübertragsspektrum von elastisch gestreuter Strahlung wesentlich von der atomaren Zusammensetzung des Körpers ab, in dem die Streustrahlung erzeugt wird. So zeigt beispielsweise das Impulsübertragsspektrum von Knochen ein Maximum bei 1,8/nm, während das Maximum von Fett bei 1,2/nm liegt. Es genügt daher, die Bestimmung des Impulsübertragsspektrums auf einen Bereich zwischen X=0,8/nm und X=2,0/nm zu beschränken. Wenn das äußerste Detektorelement $D_7$ zur Bestimmung des niedrigsten Impulsübertrages (X=0,8/nm) und das innerste Detektorelement $D_1$ zur Bestimmung des höchsten Impulsübertrages (X=2/nm) beitragen soll, dann muß die Energie der Röntgenquanten im Primärstrahl 3 etwa zwischen 30 keV und 130 keV liegen. Dies setzt voraus, daß die Röntgenröhre mit einer Spannung betrieben wird, die größer ist als 130 kV, beispielsweise 160 kV.

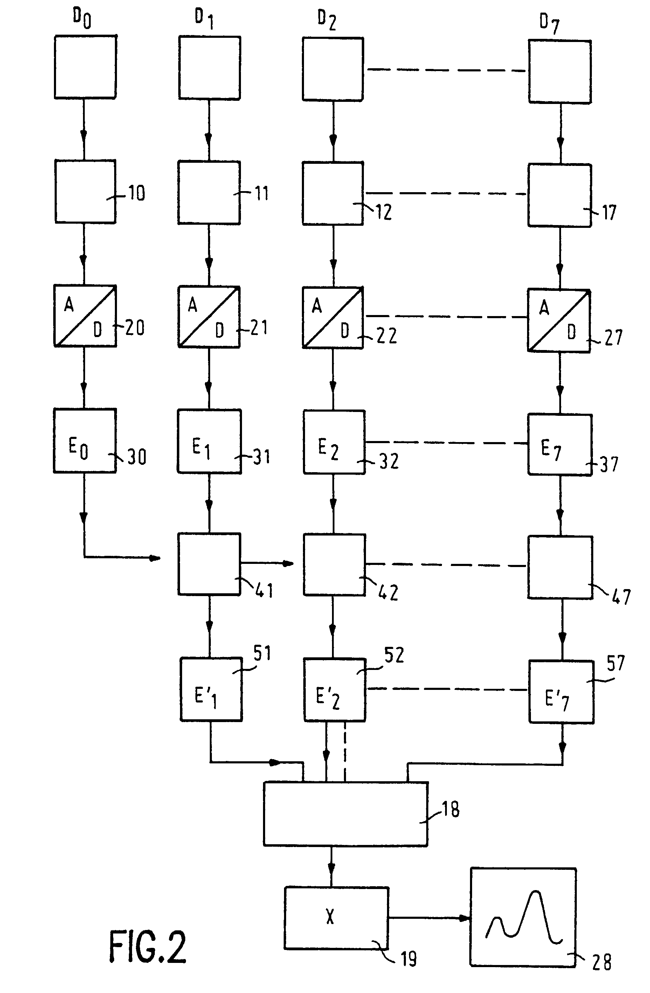

Fig. 2 zeigt schematisch das Blockschaltbild einer Anordnung zur Bestimmung des Impulsübertrages aus den Ausgangssignalen des Detektors. Danach ist dem zentralen Detektorelement $D_0$ und jedem der ringförmigen Detektorelemente $D_1$, $D_2..D_7$ ein Verstärker 10 bzw. 11, 12..17 nachgeschaltet, der das Ausgangssignal des zugeordneten Detektorelements verstärkt. Vorzugsweise sind die Verstärkungsfaktoren aller Verstärker einander gleich. Die Amplitude des Ausgangssignals der Detektorelemente ist der Energie des Röntgenquants proportional, das das betreffende Ausgangssignal hervorgerufen hat. Mithin ist auch die Amplitude der Ausgangssignale der Verstärker 10..17 der Energie der Röntgenquanten proportional.

Die analogen Ausgangssignale der Verstärker 10 bzw. 11, 12..17 werden mit Hilfe von Analog-Digital-Wandlern 20 bzw. 21, 22..27 in digitale Ausgangssignale umgesetzt derart, daß für jedes Röntgenquant der vom Digital-Analog-Wandler gelieferte Digitalwert der Amplitude des Detektor-Ausgangssignales entspricht. Zu diesem Zweck können die Digital-Analog-Wandler beispielsweise jeweils einen Kondensator enthalten, der über einen Spitzenwert-Gleichrichter durch das impulsartige Ausgangssignal des vorgeschalteten Verstärkers aufgeladen wird, und der anschließend durch einen konstanten Strom wieder vollständig entladen wird. Die Entladungsdauer ist dabei proportional zur Energie des Röntgenquants. Sie kann mittels eines elektronischen Zählregisters gemessen werden, das während der Entladungsdauer die Impulse eines Oszillators mit konstanter Frequenz zählt, wobei der Beginn der Kondensatorentladung noch mit den Oszillatorimpulsen synchronisiert wird. Die Auslösesignale für die Digital-Analog-Wandler 20, 21..27 werden dabei von den Ausgangssignalen der vorgeschalteten Detektoren abgeleitet.

Die Analog-Digital-Wandler 20 bzw. 21, 22..27 bilden zusammen mit den ihnen nachgeschalteten Schaltungen 30 bzw. 31, 32.. 37 Impulshöhenanalysatoren, die die Häufigkeitsverteilung der Amplituden der Detektorausgangssignale registrieren. Da die Amplituden der Detektorausgangssignale der Energie der Röntgenquanten proportional sind, liefern die Impulshöhenanalysatoren somit jeweils das Energiespektrum der auf die einzelnen Detektorelemente $D_0$, $D_1$..$D_7$ auftreffenden Röntgenstrahlung.

Zu diesem Zweck können die Schaltungen 30...37 jeweils einen Speicher und einen Addierer enthalten. Jedesmal, wenn einer der Analog-Digital-Wandler 20..27 einen Spannungsimpuls in ein digitales Datenwort umgesetzt hat, wird die diesem Datenwort entsprechende Adresse in dem Speicher aufgerufen und der zugehörige Addierer aktiviert. Dieser addiert zu dem Inhalt des aufgerufenen Speicherplatzes eine 1 und schreibt das Ergebnis in dem betreffenden Speicherplatz zurück, so daß dessen Inhalt im Ergebnis um 1 erhöht wird. Die Speicherplätze eines jeden Speichers enthalten am Ende einer Röntgenuntersuchung die Zahl der Röntgenquanten in dem diesem Speicherplatz zugeordneten Energiebereich, so daß auf diese Weise das Energiespektrum für jedes Detektorelement getrennt gespeichert wird.

Die Energiebereiche, in denen die Zahl der auftreffenden Röntgenquanten bestimmt wird, sollten dabei so fein unterteilt sein, daß die Genauigkeit, mit der das Spektrum bestimmt werden kann, nur durch das Energieauflösungsvermögen der Detektorelemente bestimmt wird. Wenn beispielsweise die Detektorelemente Energieunterschiede von 300 eV sicher unterscheiden können, soll die Breite der einzelnen Energiebereiche, in denen registriert wird, schmaler sein als 300 eV. Wenn die niedrigste zu registrierende Energie bei 30 keV und die höchste bei 130 keV liegt, bedeutet dies, daß z.B. 500 (oder 1000) Energiebereiche vorhanden sein sollen, und daß eine entsprechende Anzahl von Speicherplätzen in jedem Impulshöhenanalysator vorhanden sein muß.

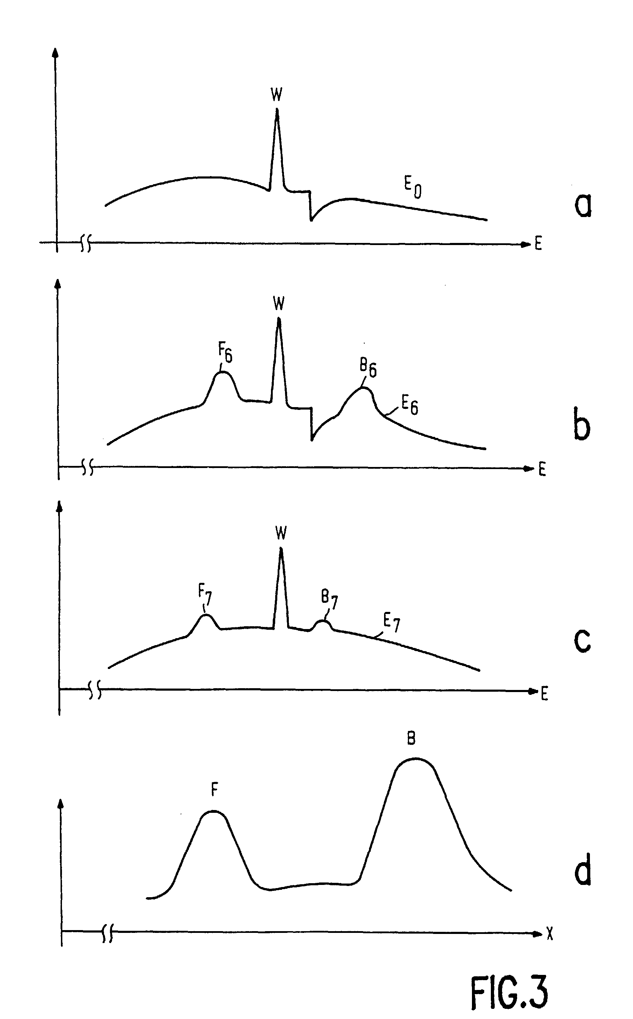

In Fig. 3a, 3b, 3c sind die Energiespektren - d. h. die Zahl der Röntgenquanten als Funktion in ihrer Energie - für verschiedene Detektorelemente dargestellt.

Fig. 3a zeigt das vom zentralen Detektorelement $D_0$ registrierte Energiespektrum $E_0$. Dieses wird einerseits durch das Emissionsspektrum des Röntgenstrahlers und andererseits durch die Energieabhängigkeit der Schwächung der Röntgenstrahlung durch das Objekt 4 bestimmt (energiearme Röntgenquanten werden stärker geschwächt als energiereiche). Man erkennt eine ausgeprägte Linie W, die von der charakteristischen Strahlung des Röntgenstrahlers hervorgerufen wird, sowie ein (relatives) Minimum, das durch die Absorptionskante des Röntgenstrahlers verursacht wird. Wenn der Röntgenstrahler eine Wolframanode enthält, liegt die charakteristische Linie W bei ca. 59 keV und die Absorptionskante bei ca. 69 keV.

Fig. 3b zeigt das Energiespektrum $E_6$ der Röntgenquanten, die auf das Detektorelement $D_6$ treffen. Einerseits ergeben sich ähnliche Strukturen wie im Energiespektrum $E_0$ (Fig. 3a), andererseits treten diesem Spektrum zusätzlich zwei (relative) Maxima $F_6$ und $B_6$ auf, die vergleichsweise flach sind. Das Maximum $F_6$ (bei ca. 47 keV) wird durch die Fettanteile im Punkt 7 (Fig. 1) verursacht, während das Maximum $B_6$ (bei ca. 72 keV) von dem Knochenanteil herrührt.

Fig. 3c zeigt das Energiespektrum $E_7$ des Detektorelementes 7, das einen größeren Radius hat wie das Detektorelement 6 und somit die Streustrahlung aus dem Punkt 7 unter einem größeren Streuwinkel empfängt. Auch hier sind wiederum zwei Maxima $F_7$ und $B_7$ nachweisbar, doch sind diese im Vergleich zum Spektrum $E_6$ zu niedrigeren Energien hin verschoben.

Um aus den Energiespektren, die für die einzelnen Detektorelemente registriert werden, ein gemeinsames Impulsübertragsspektrum registrieren zu können, muß zunächst die die Energieabhängigkeit der vom Röntgenstrahler emittierten Strahlung und der Schwächung der Strahlung durch das Untersuchungsobjekt 4 korrigiert werden. Dabei wird die Tatsache ausgenutzt, daß die Streustrahlung, die die Detektorelemente $D_1$..$D_7$ erreicht, wegen der vergleichsweise geringen Streuwinkel (2-4°) durch das Untersuchungsobjekt 4 nahezu in der gleichen Weise geschwächt werden wie der Primärstrahl 3, dessen Intensität hinter dem Untersuchungsobjekt durch das zentrale Detektorelement $D_0$ registriert wird. Auch die Energieabhängigkeit der von dem Röntgenstrahler 1 emittierten Strahlung manifestiert sich im Primärstrahl in gleicher Weise wie in der Streustrahlung. Normiert man daher die Spektren $E_1$.. $E_7$ jeweils auf das Spektrum $E_0$, indem man für jeden Energiebereich die Zahl der von dem betreffenden Detektorelement registrierten Röntgenquanten durch die Zahl der vom Detektorelement $D_0$ in demselben Energiebereich registrierten Röntgenquanten dividiert, dann wird die geschilderte Energieabhängigkeit eliminiert - und gleichzeitig auch etwaige Änderungen der bei einer Untersuchung registrierten Strahlungsdosis hinter dem Objekt.

Die geschilderte Korrektur erfolgt mit Hilfe von Korrektureinheiten 41, 42..47 die den Impulshöhenanalysatoren 21, 31..27, 37 nachgeschaltet sind, worin jede Zahl, die in einem Speicherplatz eines der den Detek-

torelementen $D_1..D_7$ zugeordneten Impulshöhenanalysators gespeichert ist, durch die Zahl dividiert wird, die in der Schaltung 30 an dem betreffenden Speicherplatz gespeichert ist.

Neben der geschilderten Korrektur können die Korrektureinheiten 41..47 weitere Korrekturen durchführen, beispielsweise die Korrektur der Hintergrundstrahlung. Diese Hintergrundstrahlung wird im wesentlichen durch die Kollimatoren hervorgerufen. Sie kann einmal bestimmt werden, indem das Energiespektrum der Streustrahlung bestimmt wird, wenn sich ein Modellobjekt im Primärstrahl 3 befindet, das Punkt 7 und seine unmittelbare Umgebung nicht ausfüllt. Es wird gespeichert und kann bei folgenden Messungen subtrahiert werden - zweckmäßigerweise vor der Normierung auf das Spektrum des zentralen Detektors.

Die auf diese Weise ermittelten korrigierten Spektren $E_1'..E_7'$ werden in je einem Speicher 51, 52..57 gespeichert. Sie entsprechen dem Spektrum, das sich ergeben würde, wenn der Röntgenstrahler ein "weißes" Spektrum emittieren würde (mit konstanter, von der Energie unabhängiger Intensität), und wenn im Strahlengang nur der Punkt 7 des Untersuchungsobjektes 4 vorhanden wäre, nicht aber der gesamte übrige Teil des Untersuchungsobjektes und auch nicht die Kollimatoranordnung 6.

Zwischen dem Impulsübertrag X gemäß Gleichung (1) und der Energie E eines unter dem Streuwinkel T gestreuten Röntgenquants besteht die Beziehung

$$X = c * E * \sin (T/2) \qquad (2)$$

wobei c eine Konstante ist. Wenn die Energie E in keV angegeben wird und der Impulsübertrag X in 1/nm, dann hat c den Wert 0,8066. Aus dieser Gleichung ergibt sich - ebenso wie aus den Figuren 3b und 3c - daß die Energie, die einem bestimmten Impulsübertrag zugeordnet ist, umso größer ist, je kleiner der Streuwinkel ist, d. h. je weiter innen das Detektorelement liegt. Die Energien, die demselben Impulsübertrag zugeordnet sind, z. B. $F_6$ und $F_7$ in Fig. 3b und 3c, verhalten sich in guter Näherung umgekehrt proportional zu dem Streuwinkel, unter dem die betreffenden Detektorelemente von Streustrahlung getroffen werden.

Die Berechnungen gemäß Gleichung (2) werden von einem Rechner 18 durchgeführt, beispielsweise einem Mikrocomputer. Sie ergeben für jedes Streuspektrum E' entsprechend des ihm zugeordneten Streuwinkels T eine bezüglich der Stützstellen unterschiedliche Impulsübertragsskala X. Eine Addition der Streuspektren erfolgt daher erst nach einer mittels Interpolation ausgeführten Umrechnung auf einheitliche Stützstellen in der X-Skala. Die Addition der Spektren wird gewichtet vorgenommen mit vorher einmalig empirisch ermittelten Gewichtungen. Sie ist im allgemeinen notwendig, um die unterschiedliche Qualität der Einzelspektren zu berücksichtigen. Das so ermittelte Impulsspektrum wird in einem Register 19 gespeichert und steht damit einer weiteren Auswertung zur Verfügung. Es kann auf einer geeigneten Wiedergabeeinheit 28 wiedergegeben werden.

Figur 3d zeigt ein Beispiel eines solchen Impulsübertragsspektrums. Ein solches Impulsübertragsspektrum kann dann resultieren, wenn der Untersuchungspunkt 7 sich in der spongiösen Knochensubstanz, vorzugsweise der Wirbelsäule, befindet. Dieses Impulsübertragsspektrum zeigt ein Maximum F (bei ca. 1,2/nm), das von dem Fettanteil im Punkt 7 herrührt, und ein Maximum B, das von dem Knochenanteil herrührt und bei ca. 1,8/nm liegt. Der Quotient aus B und F bzw. aus den Flächen unterhalb von B und F ist ein gutes Maß für den Mineralgehalt bzw. die Festigkeit eines Knochens. Je höher dieser Quotient ist, desto besser ist der Zustand des Knochengewebes.

Der Mikrocomputer 18 kann außer der Berechnung des Impulsübertrages noch weitere Funktionen übernehmen, beispielsweise die Korrektur der Energiespektren. In diesem Fall wären die Korrekturschaltungen 41..47 durch eine entsprechende Programmierung des Mikrocomputers ersetzt.

Wenn die Streustrahlung der Struktur in dem Untersuchungspunkt keine Vorzugsrichtung hat, ergibt sich ein rotationssymmetrisches Beugungsmuster. Wenn dies jedoch nicht der Fall ist, kann es von Interesse sein, die elastische Streustrahlung als Funktion der Azimultalkomponente zu messen. In diesem Fall müßte jeder der Detektorringe $D_1..D_7$ in mehrere - z.B. acht - bogenförmige Segmente unterteilt sein, wobei eine höhere Anzahl von Verarbeitungskanälen (z.B. 11.. 51) vorhanden sein müßte. Ein entsprechend unterteilter Detektorring ist in Fig. 1b dargestellt. Da diametral gegenüberliegende Segmente aber in der Regel derselben Streuintensität ausgesetzt sind, wären diese Schaltungen nur für jeweils zwei Segmente erforderlich.

Statt der Unterteilung der Detektorringe könnte vor dem Detektor D auch eine Blende angeordnet sein, die jeweils nur zwei gegenüberliegende Sektoren der Ringe freiläßt, so daß nur in diesen Sektoren Streustrahlung gemessen werden kann. Anschließend könnte die Blende gedreht werden, so daß andere Sektoren von Streustrahlung getroffen würden. Hierbei wird die dem Untersuchungsobjekt 4 zugeführte Dosis vergrößert; allerdings wäre die Anzahl der Verarbeitungskanäle kleiner als bei gemäß Fig. 1b unterteilten Detektorelementen.

Vorstehend wurde die Erfindung im Zusammenhang mit eienr medizinisch-diagnostischen Anwendung erläutert. Sie ist aber grundsätzlich auch auf anderen Gebieten, z.B. der Materialuntersuchung, anwendbar.

**Patentansprüche**

1. Röntgengerät mit einem polychromatischen Röntgenstrahler (1) zur Erzeugung eines Primärstrahls (3) von geringem Querschnitt, einer energieauflösend messenden Detektoranordnung (D) zur Erfassung der durch elastische Streuprozesse im Primärstrahl erzeugten Streustrahlung mit mehreren Detektorelementen, die auf zum Primärstrahl konzentrischen Ringen angeordnet sind, einer zwischen dem Röntgenstrahler und der Detektoranordnung angeordneten, den Primärstrahl umschließenden Kollimatoranordnung (6) und einer an die Detektoranordnung angeschlossenen Auswerteeinrichtung,
dadurch gekennzeichnet, daß die Kollimatoranordnung (6) so gestaltet ist, daß die Detektorelemente $(D_1....D_7)$ von der Streustrahlung nur ein und desselben punktförmigen Abschnitts (7) des Primärstrahls getroffen werden, und daß die Auswerteeinrichtung aus den Ausgangssignalen der Detektorelemente $(D_1...D_7)$ das Impulsübertragsspektrum in dem punktförmigen Abschnitt ermittelt.

2. Röntgengerät nach Anspruch 1,
dadurch gekennzeichnet, daß für jedes Detektorelement $(D_1..D_7)$ Mittel $(11..51, 17..57)$ zum Registrieren des Energiespektrums der auf das betreffende Detektorelemente auftreffenden Röntgenquanten vorgesehen sind.

3. Röntgengerät nach Anspruch 2,
dadurch gekennzeichnet, daß die Detektoranordnung ein im Primärstrahl befindliches zentrales Detektorelement $(D_0)$ umfaßt, daß Mittel $(10...30)$ zum Registrieren des $(E_0)$ Energiespektrums des zentralen Detektorelements $(D_0)$ vorgesehen sind sowie Mittel zum Normieren der Energiespektren $(E_1..E_7)$ der anderen Detektorelemente $(D_1..D_7)$ auf das Energiespektrum $(E_0)$ des zentralen Detektorelements $(D_0)$.

4. Röntgengerät nach Anspruch 2 oder 3,
dadurch gekennzeichnet, daß Mittel (18) zum Bestimmen des Impulsübertragsspektrums (X) aus den gegenbenenfalls normierten Energiespektren $(E_1'..E_7')$ der das zentrale Detektorelement umschließenden Detektorelemente vorgesehen sind.

5. Röntgengerät nach einem der Ansprüche 1 bis 4,
dadurch gekennzeichnet, daß die Kollimatoranordnung (6) mehrere konzentrisch zum Primärstrahl angeordnete Kollimatorlamellen umfaßt, die die Form eines Kegelstumpf-Mantels haben und deren Verlängerungen sich in demselben Punkt (7) des Primärstrahls (3) schneiden.

**Claims**

1. An X-ray apparatus, including a polychromatic X-ray source (1) for generating a primary beam (3) of small cross-section, an energy-sensitive detector arrangement (D) for detecting the scattered radiation generated by elastic scattering processes in the primary beam path, which detector arrangement comprises a plurality of detector elements which are arranged on rings concentric with the primary beam, a collimator arrangement (6) which is arranged between the X-ray source and the detector arrangement and encloses the primary beam and an evaluation device which is connected to the detector arrangement, characterized in that the collimator arrangement (6) is constructed so that only the scattered radiation from one and the same point-shaped section (7) of the primary beam is incident on the detector elements $(D_1 ... D_7)$ and that the evaluation device determines the pulse transfer spectrum in the oin-shaped section from the output signals of the detector elements $(D_1...D_7)$.

2. An X-ray apparatus as claimed in Claim 1, characterized in that for each detector element $(D_1 ... D_7)$ there are provided means $(11 ... 51, 17 ... 57)$ for recording the energy spectrum of the X-ray quanta incident on the relevant detector element.

3. An X-ray apparatus as claimed in Claim 2, characterized in that the detector arrangement comprises a central detector element $(D_0)$ which is situated in the primary beam, there being provided means $(10... 30)$ for recording the energy spectrum $(E_0)$ of the central detector element $(D_0)$ as well as means for normalizing the energy spectra $(E_1 ... E_7)$ of the other detector elements $(D_1 ... D_7)$ to the energy spectrum $(E_0)$ of the central detector element $(D_0)$.

4. An X-ray apparatus as claimed in Claim 2 or 3, characterized in that there are provided means (18) for determining the pulse transfer spectrum (X) from the possibly normalized energy spectra $(E_1' ... E_7')$ of the detector elements enclosing the central detector element.

5. An X-ray apparatus as claimed in one of the Claims 1 to 4, characterized in that the collimator arrangement (6) comprises a plurality of collimator laminations which are arranged so as to be concentric with the primary beam and are shaped as a truncated cone surface, their prolongations intersecting one another in the same point (7) of the primary beam (3).

**Revendications**

1. Appareil à rayons X muni d'un radiateur de rayons X polychromatique (1) pour la réalisation d'un rayon primaire (3) présentant une petite section transversale, un dispositif de détection (D) permettant la mesure à l'aide de résolution d'énergie afin de capter le rayonnement diffusé se produisant par suite de processus de diffusion élastiques dans le rayon primaire à l'aide de plusieurs éléments de détection, qui sont disposés sur des anneaux concentriques par rapport au rayon primaire, un dispositif de collimation (6) disposé entre le radiateur de rayons X et le dispositif de détection et entourant le rayon primaire et un dispositif d'évaluation connecté au dispositif de détection, caractérisé en ce que le dispositif de collimation (6) est réalisé de façon que les éléments de détection $(D_1 ... D_7)$ ne soient frappés que par le rayonnement diffusé à partir d'une seule et même section ponctuelle (7) du rayon primaire et que le dispositif d'évaluation détermine le spectre de transmission d'impulsions dans la section ponctuelle à partir des signaux de départ provenant des éléments de détection $(D_1 ...D_7)$.

2.    Appareil à rayons X selon la revendication 1, caractérisé en ce que pour chaque élément de détection $(D_1 ... D_7)$ sont disposés des moyens (11..51, 17.. 57) pour enregistrer le spectre d'énergie des quanta de rayons X parvenant sur les éléments de détection en question.

3.    Appareil à rayons X selon la revendication 2, caractérisé en ce que le dispositif de détection comprend un élément de détection central $(D_o)$ se trouvant dans le rayon primaire, que des moyens (10 ...30) sont prévus pour enregistrer le spectre d'énergie $(E_o)$ de l'élément de détection central $(D_o)$, ainsi que des moyens pour normaliser les spectres d'énergie $(E_1..E_7)$ des autres éléments de détection $(D_1..D_7)$ sur le spectre d'énergie $(E_o)$ de l'élément de détection central.

4.    Appareil à rayons X selon la revendication 2 ou 3, caractérisé en ce que des moyens (18) sont prévus pour déterminer le spectre de transmission d'impulsions (X) à partir des spectres d'énergie normalisés le cas échéant $(E_1'..E_7')$ des éléments de détection entourant l'élément de détection central.

5.    Appareil à rayons X selon l'une des revendications 1 à 4, caractérisé en ce que le dispositif de collimation (6) comprend plusieurs lamelles collimatrices, qui sont disposées d'une façon centrique par rapport au rayon primaire et qui présentent la forme d'une enveloppe tronconique et dont les prolongements se coupent dans le même point du rayon primaire (3).

a

b

FIG.1

FIG.2

FIG.3